# EUROPEAN PATENT SPECIFICATION

(11) **EP 3 651 164 B1**
(45) Date of publication and mention of the grant of the patent: **26.08.2026**
(21) Application number: 18209230.4
(22) Date of filing: 29.11.2018
(51) Int. Cl.: G16H 50/20, G16H 50/30, G16H 50/70

(54) **METHOD AND SYSTEM OF DETERMINING A PROBABILITY OF A BLOOD GLUCOSE VALUE FOR A PATIENT BEING IN AN ADVERSE BLOOD GLUCOSE RANGE AT A PREDICTION TIME, AND COMPUTER PROGRAM PRODUCT**
VERFAHREN UND SYSTEM ZUR BESTIMMUNG DER WAHRSCHEINLICHKEIT EINES BLUTZUCKERWERTS FÜR EINEN PATIENTEN, DER SICH ZU EINER VORHERSAGEZEIT IN EINEM UNERWÜNSCHTEN BLUTZUCKERBEREICH BEFINDET, UND COMPUTERPROGRAMMPRODUKT
PROCÉDÉ ET SYSTÈME PERMETTANT DE DÉTERMINER LA PROBABILITÉ QU'UNE VALEUR DE GLUCOSE SANGUIN D'UN PATIENT SE TROUVANT DANS UNE PLAGE DE GLUCOSE SANGUIN DÉFAVORABLE À UN MOMENT DE PRÉDICTION ET PRODUIT-PROGRAMME D'ORDINATEUR

(30) Priority: 07.11.2018 US 201862756630 P
(43) Date of publication of application: 13.05.2020
(73) Proprietor: Roche Diabetes Care GmbH, 68305 Mannheim (DE); F. Hoffmann-La Roche AG, 4070 Basel (CH); mySugr GmbH, 1010 Wien (AT)
(72) Inventor: Duke, David L., 68305 Mannheim (DE); Wrede, Jan, 68305 Mannheim (DE); Bankosegger, Rafael, 68305 Mannheim (DE)
(74) Representative: Bittner, Thomas L.

(56) References cited:
- WO-A1-2018/153648
- US-A1- 2007 282 180
- US-A1- 2011 313 674
- US-A1- 2015 190 098
- US-A1- 2018 272 063

## Description

The present disclosure refers to a method for determining a probability of a blood glucose value for a patient being in an adverse blood glucose range at a prediction time. Further, the present disclosure refers to a system for determining a probability of a blood glucose value for a patient being in an adverse blood glucose range at a prediction time. Also, a computer program product is referred to.

### Background

Therapy improvements for diabetics can often be achieved by focusing on problematic time periods such as hyperglycemic events in the morning. Retrospective analysis of the blood glucose measurements can reveal these periods by evaluating the probabilities for glycemic excursions through the course of a day. For patients using a continuous glucose monitoring (CGM) device, the measurement frequency (1 / T) is very high (with measurement time interval T approximately ranging from 5 to 15 minutes), which allows analyses with high resolution. Here, the Ambulatory Glucose Profile has shown great acceptance throughout diabetes care society (Danne, et al., Diabetes Care (2017) 40). In contrast, for patients performing traditional (spot monitoring) blood glucose measurements (BGM), the number of samples per day is comparably low (e.g., around 3 to 8 samples) and mostly unevenly distributed in time.

To still obtain feasible statistics for BGM performing patients, measurements are to be grouped (binned) into rather large time slots (hourly or quarter-daily), which consequently leads to a loss of resolution. Sparse BGM data usually provides limited insight into a patient's glucose dynamics and corresponding risks of adverse events such as hypoglycemia and hyperglycemia.

Document US 2011/0313674 A1 refers to a testing method for optimizing a therapy to a diabetic patient, the method comprising: collecting at least one sampling set of biomarker data, and computing a probability distribution function, a hazard function, a risk function, and a risk value for the sampling set of biomarker data. The probability distribution function is calculated to approximate the probability distribution of the biomarker data. The hazard function is a function which yields higher hazard values for biomarker readings in the sampling set indicative of higher risk of complications.

US 2015/0190098 A1 discloses an adaptive advisory control interactive process involving algorithm-based assessment and communication of physiologic and behavioral parameters and patterns assisting patients with diabetes with the optimization of their glycemic control. The method and system uses sources of information about the patient; (i) EO Data (e.g. self-monitoring of blood glucose (SMBG) and CMG), (ii) Insulin Data (e.g. insulin pump log files or patient treatment records), and (iii) Patient Self Reporting Data (e.g. self treatment behaviours, meals, and exercise) to retroactively assess the risk of hypoglycemia, retroactively assess risk-based reduction of insulin delivery, and then to report to the patient how a risk-based insulin reduction system would have acted consistently to prevent hypoglycemia.

WO 2018/153648 A1 discloses systems and methods for communicating a dose history configured for representing a central tendency and a variability of a distribution of injections with a blood glucose regulating medicament. The device is adapted for performing the method of obtaining one or more qualified groups of injection events within a distribution of injection events, wherein each qualified group of injection events comprises a group-time indicator.

US 2018/0272063 A1 refers to an infusion device, a patient data management system, and a method for monitoring a physiological condition of a patient. The infusion device includes an actuation arrangement operable to deliver fluid to a user, a communication interface to receive measurement data indicative of a physiological condition of the user, a sensing arrangement to obtain contextual measurement data, and a control system coupled to the actuation arrangement.

US 20070/0282180 A1 discloses a device for measuring the glucose level in a living body comprising an electrode arrangement to be applied to a surface of the body. The glucose level is derived from the response of the electrode arrangement to an electrical signal.

### Summary

It is an object of the present disclosure to provide a method and a system for determining, for a blood glucose value for a patient, a probability of being in an adverse blood glucose range at a prediction time, wherein the probability can be determined with high accuracy based on spot monitoring blood glucose measurements.

For solving the object, a method of determining, for a blood glucose value for a patient, a probability of being in an adverse blood glucose range at a prediction time according to the independent claim 1 is provided. Further, a system for determining, for a blood glucose value for a patient, a probability of being in an adverse blood glucose range at a prediction time according to the independent claim 11 is provided. Still, claim 12 refers to a computer program product.

According to an aspect, a method of determining a probability of a blood glucose value or level for a patient being in an adverse blood glucose range at a prediction time is provided. The method, in a system having one or more data processors, comprises providing spot monitoring blood glucose measurement data representing a plurality of blood glucose measurement values for a measurement time period, wherein the blood glucose measurement values comprise first blood glucose measurement values assigned to a first adverse range of blood glucose values, and second blood glucose measurement values assigned to a second adverse range of blood glucose values, wherein the second range of blood glucose values is different from the first adverse range of blood glucose values. By applying an analysis algorithm comprising a kernel density estimation and application of Bayes' rule, determining from the spot monitoring blood glucose measurement data comprising the first blood glucose measurement values and the second blood glucose measurement values, a probability of the blood glucose value of a patient being in the first adverse blood glucose range at a prediction time, and the blood glucose value of the patient being in the second adverse blood glucose range at a prediction time. In the kernel density estimation, a first kernel bandwidth is applied for all or some of the first blood glucose measurement values, and a second kernel bandwidth different from the first kernel bandwidth is applied for all or some of the second blood glucose measurement values. Output data indicative of the prediction time and the probability value determined at the prediction time are provided.

According to another aspect, a system for determining a probability of a blood glucose value or level for a patient being in an adverse blood glucose range at a prediction time is provided. The system is having one or more data processors configured to perform the method of determining the probability of the blood glucose value or level for the patient being in an adverse blood glucose range at a prediction time.

Further, a computer program product is provided, comprising program code configured to, when loaded into a computer having one or more processors, perform the method of determining, for a blood glucose value for a patient, a probability of being in an adverse blood glucose range at a prediction time.

The spot monitoring blood glucose measurement data may be provided for a single patient. The measurement time period, for example, may extend over at least 24 hours (day). For example, the spot monitoring blood glucose values may be covering a measurement time period of 14 days with at least for 4 spot monitoring values per day on the average. Spot monitoring blood glucose measurement data for several days may be taken into account for determining the probability. The spot monitoring blood glucose measurement data may be preprocessed, e.g. by averaging blood glucose measurement values.

The first bandwidth may be applied for all or a subset of the first blood glucose measurement values in the kernel density estimation. In addition or alternatively, the second bandwidth may be applied for all or a subset of the second blood glucose measurement values in the kernel density estimation.

The output data may be outputted by an output device of the system such as a display.

The system may be implemented in a device selected from the following group: mobile or cell phone, handheld computer device, and laptop.

The kernel density estimation applied for determining the time-dependent probability from the spot monitoring blood glucose measurements is a non-parametric way to estimate the probability density function of a random variable, i.e. the spot monitored blood glucose measurement values.

In response to finding the predicted blood glucose value to be (with high likelihood, e.g. a likelihood of more than about 50%) in one of the different adverse ranges of blood glucose values, a response message may by generated by the system and outputted to the patient or user. For example, it may be proposed to the patient to have some carbohydrate intake or some insulin dosage applied in advance with respect to some likely upcoming adverse event (blood glucose values likely to be in one of the adverse ranges of blood glucose values).

The first and the second adverse blood glucose range may refer to hypoglycemia and hyperglycemia, respectively.

In an example, the first and second adverse range may refer to sub-ranges of the hypoglycemia range or class (e.g., both sub-ranges referring to blood glucose values being below or equal to 70 mg / dl), but covering different sub-ranges of hypoglycemia. In another example, the first and second adverse range may refer to sub-ranges of the hyperglycemia range or class (e.g., both sub-ranges referring to blood glucose values being equal to or above 180 mg / dl), but covering different sub-ranges of hyperglycemia.

The probability may be determined for a plurality of prediction times in a prediction period of time from the spot monitoring blood glucose measurement data. For example, a prediction period of time 24 hours may be applied.

A continuous course of the probability may be determined for the prediction period of time from the spot monitoring blood glucose measurement data. A continuous probability curve extending over the prediction period of time may be determined. Such continuous course or curve is determined from the non-continuously (discretely) measured spot monitoring blood glucose values.

In the method, the applying of the analysis algorithm, specifically the kernel density estimation, may comprise determining the probability of the blood glucose value of the patient being in the first adverse blood glucose range from a first measurement data subset of the spot monitoring blood glucose measurement data comprising at least the first blood glucose measurement values assigned to the first adverse range of blood glucose values. Determining of the probability of the blood glucose value of the patient being in the first adverse blood glucose range may be accomplished by applying the kernel density estimation to the first blood glucose measurement values only. The determining of the probability of the blood glucose value of the patient being in the first adverse blood glucose range may comprise determining whether the blood glucose values indicate hypoglycemia. For example, blood glucose values being below or equal to 70 mg / dl may be considered indicating hypoglycemia.

The applying of the of the analysis algorithm, specifically the kernel density estimation, may comprise determining the probability of the blood glucose value of the patient being in the second adverse blood glucose range from a second measurement data subset of the spot monitoring blood glucose measurement data comprising at least the second blood glucose measurement values assigned to the second adverse range of blood glucose values. The determining of the probability of the blood glucose value of the patient being in the second adverse blood glucose range may be accomplished by applying the kernel density estimation to the second blood glucose measurement values only. The determining of the probability of the blood glucose value of the patient being in the second adverse blood glucose range may comprise determining whether the blood glucose values indicate hyperglycemia. For example, blood glucose values being equal to or above 180 mg / dl may be considered indicating hyperglycemia.

The determining of the probability may comprise determining a probability for the blood glucose value of the patient being in a non-adverse blood glucose range for the prediction time, wherein the non-adverse blood glucose range is different from the first and second adverse blood glucose range. The kernel bandwidth applied for determining such probability may be different from at least one of the first kernel bandwidth, and the second kernel bandwidth. The determining of the probability of the blood glucose value of the patient being in the non-adverse blood glucose range may refer to determining whether the blood glucose values are outside blood glucose value ranges not indicating one of hyperglycemia and hypoglycemia. For example, blood glucose values being between 70 mg / dl and 180 mg / dl may be considered indicating the non-adverse range.

In still another example, the determining of the probability may comprise determining a probability for the blood glucose value of the patient being in a further blood glucose range for the prediction time, wherein the further or additional blood glucose range which may also be referred to as unknown range is different from all the non-adverse blood glucose range, the first adverse blood glucose range, and the second adverse blood glucose range. The kernel bandwidth applied for determining such probability may be different from at least one of the first kernel bandwidth, the second kernel bandwidth, and the kernel bandwidth applied in the determining the probability for the blood glucose value being in the non-adverse blood glucose range.

The determining may further comprise applying a third kernel bandwidth in the kernel density estimation which is different from both the first and the second kernel bandwidth.

In the method, the applying may comprise applying a periodic kernel in the kernel density estimation,

The first kernel bandwidth may be broader than the second kernel bandwidth. For example, the first kernel bandwidth may be broader by a factor of about 1.2 to about 2.0.

The applying may comprise the following: applying a first bandwidth value for a measurement value form the first blood glucose measurement values, and applying a second bandwidth value for a further measurement value from the first blood glucose measurement values, wherein the first bandwidth value is different front from the second bandwidth value. In this example there is not only bandwidth variation or adaption for the first and second blood glucose measurement values assigned to the first and second adverse range, respectively, but also for different first blood glucose measurement values assigned to the first adverse range. The first and second bandwidth values may be different from at least one of the second kernel bandwidth and the third kernel bandwidth.

Similarly, the following may be provided alternatively or in addition: applying a first bandwidth value for a measurement value form the second blood glucose measurement values, and applying a second bandwidth value for a further measurement value from the second blood glucose measurement values, wherein the first bandwidth value is different front from the second bandwidth value.

In an alternative example single bandwidth value is applied for all first blood glucose measurement values assigned to the first adverse range of blood glucose values on one side, and / or all second blood glucose measurement values assigned to a second adverse range of blood glucose values on the other side.

The following may be provided: The first adverse range of blood glucose values is assigned to blood glucose measurement values indicative of a hypoglycemic state for the patient, and the second adverse range of blood glucose values is assigned to blood glucose measurement values indicative of a hyperglycemic state for the patient.

The embodiments disclosed above for the method may apply to the system *mutatis mutandis.*

With the focus in diabetes care turning towards increasing the time in range, the time during which a patient's blood glucose level is in a non-adverse range, the proposed technology provides a robust way for estimating the time in range as well as highlighting specific times of day where a patient experiences challenges toward maintaining the time in range. High level metrics such as HbA1c or time in range provide an easy to understand metric of therapy success, but are difficult to translate directly into needed therapy adjustments. Identifying problematic times of day, i.e. the patient's blood glucose level being in an adverse range, can better be linked to therapy problems and consequently translated into concrete adjustments.

The technology further allows the identification of problematic time of day periods which can be used to create therapy recommendations or adjustments to the patient and shift his / her focus on a specific period of the day. Based on the time of day, there can be different underlying problems leading to the observation. This, for instance, allows the utilization of the model as part of an automated therapy recommendation system and the provision of educational material. Notifications can be generated and sent (outputted) to a patient based on the determined probability of an upcoming adverse event. Because of the method involving the kernel density estimation, the patient can be notified before the adverse event is likely to occur, which may prompt a behavior change.

The technology proposed could also drive patient specific challenges and feedback. If for a patient non-adverse blood glucose values were determined during a challenging time of day, positive reinforcement could be provided.

A comprehensive visual representation (e.g., by means of the output data) can be provided that allows a fast therapy performance assessment both for a health care provider and the patient. An entry point for BGM therapy assessment may be provided. Problematic daytimes can be revealed, which can be further investigated. Occurring changes of the probabilities of being in an adverse state can be highlighted in order to indicate potential positive or negative changes in the person's therapy. For example, if the probability for being in a hypoglycemic state changes overnight between visits, an accordant notification could be provided to the health care provider.

The resulting probability density function(s) is a continuous and do not suffer resolution problems due to binning, which could ultimately lead to the determining of inaccurate time of day periods as well as an inability to determine time of day periods with in adequate control in cases were searched time of day periods are spread over multiple bins.

Moreover, a skewness of self-monitoring of blood glucose (SMBG), also being a type spot monitoring measurement, distributions can be accounted for by normalisation through evidence on a daily basis thus, the effect of data skewness, e.g., due to an increased blood glucose measurement frequency at certain events or times of day, does not affect the determined probabilities for multiple days.

By the technology proposed, evaluation or analysis of the spot monitoring blood glucose measurement results is improved. An analysis comprising a statistical analysis as applied. The evaluation of the experimentally (by measurement) gathered results can be improved. From the spot monitoring blood glucose measurement results prediction is derived for the probability of the blood glucose level of the patient being in an adverse (or an non-adverse) range, also at times different from the measurement times at which spot monitoring blood glucose measurements have been conducted, specifically at times adjacent to the measurement times (neighboring times).

### Description of further embodiments

In the following, embodiments, by way of example, are described with reference to figures. In the figures show:

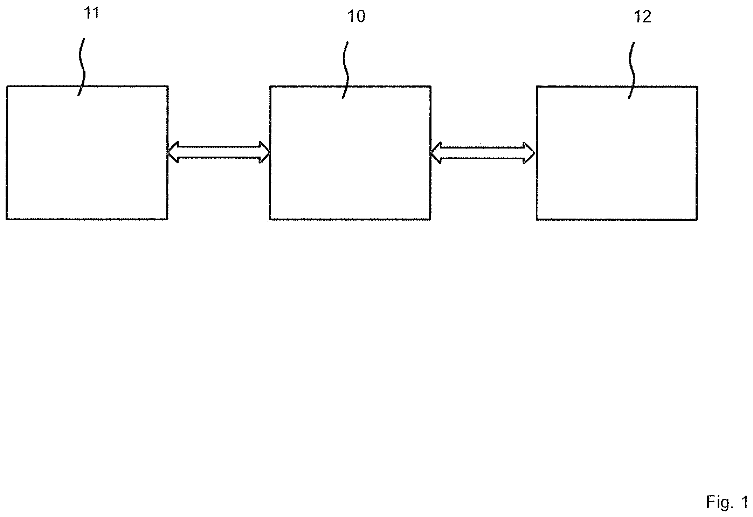

- Fig. 1: a schematic representation of an arrangement for providing spot monitoring blood glucose measurement (BGM) data and determining from such BGM data, for a blood glucose value for a patient, a probability of being in an adverse blood glucose range at a prediction time;
- Fig. 2: a graphical representation of probabilities of being in a certain class for each point in time calculated from continuous glucose monitoring (CGM) data;
- Fig. 3: a graphical representation of the discrete kernel bandwidth values together with a fitting function in the hypoglycemia class;
- Fig. 4: a graphical representation the discrete kernel bandwidth values together with a fitting function in the hyperglycemia class;
- Fig. 5: a graphical representation of estimated probability density functions (PDFs) which are estimated from discrete blood glucose measurement values;
- Fig. 6: a graphical representation of the probabilities of being in a hyperglycemic state, a hypoglycemic state, or an non-adverse blood glucose state for each time of day.
- Fig. 7: a polar representation of estimated PDFs after normalization;
- Fig. 8: a graphical representation of the probabilities of being in a hyperglycemic state, a hypoglycemic state, or a non-adverse blood glucose state determined from discrete BGM values in comparison with the probabilities determined from a CGM data set;
- Fig. 9: a graphical representation of residual sums of squared errors (RSS) as a function of the number of measurements per day;
- Fig. 10: shows a graphical representation of the probabilities of being in a hypoglycemic state, a non-adverse blood glucose state, a hyperglycemic state, or an unspecified state, determined from discrete BGM values; and
- Fig. 11: shows another graphical representation of the probabilities of being in a hypoglycemic state, a non-adverse blood glucose state, a hyperglycemic state, or an unspecified state, determined from discrete BGM values.

A method and a system for determining, for a blood glucose value for a patient, a probability of being in an adverse blood glucose range at a prediction time is provided. The method, in a system 10 having one or more data processors, comprises providing spot monitoring blood glucose measurement (BGM) data representing a plurality of blood glucose measurement values for a measurement time period, wherein the blood glucose measurement values comprise first blood glucose measurement values assigned to a first adverse range of blood glucose values, and second blood glucose measurement values assigned to a second adverse range of blood glucose values, wherein the second range of blood glucose values is different from the first adverse range of blood glucose values. The BGM data are provided to the system 10 through an input device 11 which can be connected to a spot monitoring measurement device (not shown) or a data base in which the BGM data have been stored after spot monitoring measurements. The first and second adverse range may refer to hypoglycemia and hyperglycemia, respectively.

A kernel density estimation is applied to determine, from a measurement data subset or all of the BGM data, a probability for a prediction time for the blood glucose value(s) of the patient being in the first adverse blood glucose range or in the second adverse blood glucose range. Further, a probability may be determined for the blood glucose value being in an non-adverse range.

In the kernel density estimation conducted in the system 10, a first kernel bandwidth is applied for the first blood glucose measurement values, and a second kernel bandwidth different from the first kernel bandwidth is applied for the second blood glucose measurement values. Output data indicative of the prediction time and the probability value determined for the prediction time are provided in the system 10 and outputted through an output device 12 such as a display and / or an audio device.

Probability density functions (PDF), also called probability distribution functions, can provide the probabilities of random variables, such as being in the hypoglycemic state, falling within a particular continuous range of values, such as a time range. Kernel Density Estimation (KDE) is a method for providing an estimator *f̂* for an unknown continuous probability density function (or probability distribution function) *f* based on a data set of discrete measurement values using n Kernel functions K via the formula $\hat{f} \left(t\right) = \frac{1}{n ν} {\sum }_{i = 1}^{n} K \left(\frac{t - {t}_{i}}{ν}\right) ,$ with kernel bandwidth v. The Gaussian kernel with $K \left(u\right) = \frac{1}{\sqrt{2 π}} {e}^{- \frac{1}{2} {u}^{2}}$ is a common choice for KDE problems.

In an example, the discrete measurement values are provided by the result of the spot monitoring measurement for one patient.

Operating over a 24-hour cycle, however, means that blood glucose measurement data measured just before midnight should also affect the resulting probability determined just after midnight. A traditional Gaussian kernel cannot accommodate such condition. Instead, the von Mises kernel with ${K}_{i , ν} \left(θ\right) = {e}^{ν cos \left(θ-{θ}_{i}\right)}$ is employed, which is a function of an angle *θ* with 0 ≤ *θ* ≤ 2*π*. The Mises kernel being an example for the periodic kernel type thus works in instances where modular arithmetic is needed, e.g., where the numbers wrap around upon reaching a certain value. The kernel bandwidth v is related to the variance *σ*² via the relationship $\frac{1}{ν} \approx {σ}^{2}$ for larger values of *ν*. The values *θᵢ* correspond to the positions around which each von Mises Kernel is centered, analogous to the mean *µ* of a distribution function.

The time of day t in hours, whereby *t* is from the range [0, 24], must be transformed into an angle such that -*π* ≤ *θ* ≤ *π*, which is achieved by $θ = \left(\left(2πt\right)/24\right) - π .$

The resulting estimator *f̂* for the PDF *f* has the formula $\hat{f} \left(θ\right) = \frac{1}{n \left(2π\right) {I}_{0} \left(ν\right)} {\sum }_{i = 1}^{n} {e}^{ν cos \left(θ-{θ}_{i}\right)} .$

The goal is to determine the probability of the occurrence of an event with a specific class *cⱼ* for a specific time of day, t. The classes may correspond to adverse and / or non-adverse blood glucose ranges. In particular, the classes may each correspond to a hyperglycemic state, a hypoglycemic state, and a non-adverse blood glucose state. The sparseness in time of blood glucose measurement data makes this particularly challenging.

The blood glucose measurement data can be used to calculate conditional probabilities *P*(*t*|*cⱼ*), from which the conditional probabilities *P*(*cⱼ*|*t*) are calculated by applying Bayes' rule, $P \left({c}_{j}\left|t\right.\right) = \frac{P \left(t\left|{c}_{j}\right.\right) P \left({c}_{j}\right)}{P \left(t\right)} .$

The prior probability for the class *cⱼ* can be derived from the formula $P \left({c}_{j}\right) = \frac{{n}_{j}}{N}$, with *nⱼ* being the number of blood glucose measurements in a specific class and N being the total number of blood glucose measurements.

The probability of being in a specific event class *cⱼ* for neighboring times near a given blood glucose value is dependent on the measured value of blood glucose. If a blood glucose measurement is extremely large, it is unlikely that the blood glucose value is in the non-adverse blood glucose range near that time.

A clinical CGM data set is used to determine the kernel bandwidth v for a specific glucose value. Notably, the kernel bandwidth v is adaptive with respect to the blood glucose value. To this, the PDF of each class in a time window surrounding the measurement is determined from the CGM data set.

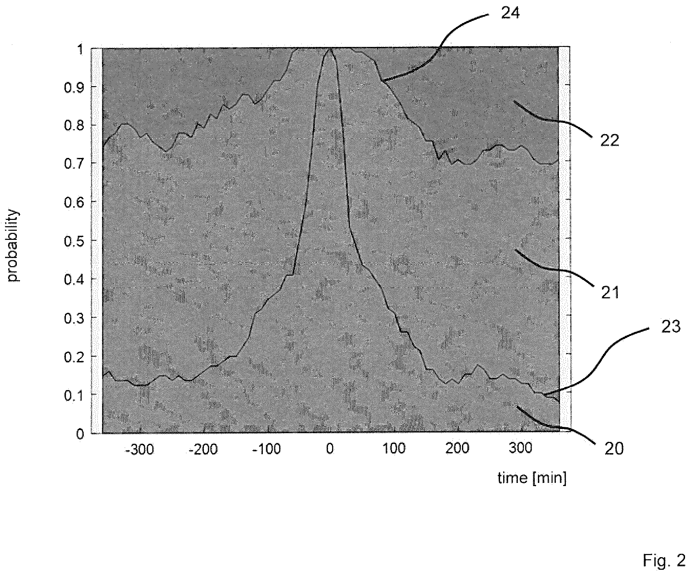

Fig. 2 shows a graphical representation of probabilities of being in a certain class for each point in time resulting from the CGM data set PDFs. Class *c*₁ corresponds to blood glucose values below or equal to 70 mg / dl (hypoglycemia) and to area 20; class *c*₂ corresponds to blood glucose values between 70 mg / dl and 180 mg / dl (non-adverse blood glucose range) and to area 21; class *c*₃ corresponds to blood glucose values equal to or above 180 mg / dl (hyperglycemia) and to area 22.

Curve 23 corresponds to the hypoglycemia PDF, curve 24 to the non-adverse blood glucose range PDF plus the hypoglycemia PDF. Since there are only the three classes *c*₁, *c*₂, *c*₃ in this example, the corresponding PDFs add up to one (corresponding to 100 % probability) for each point in time.

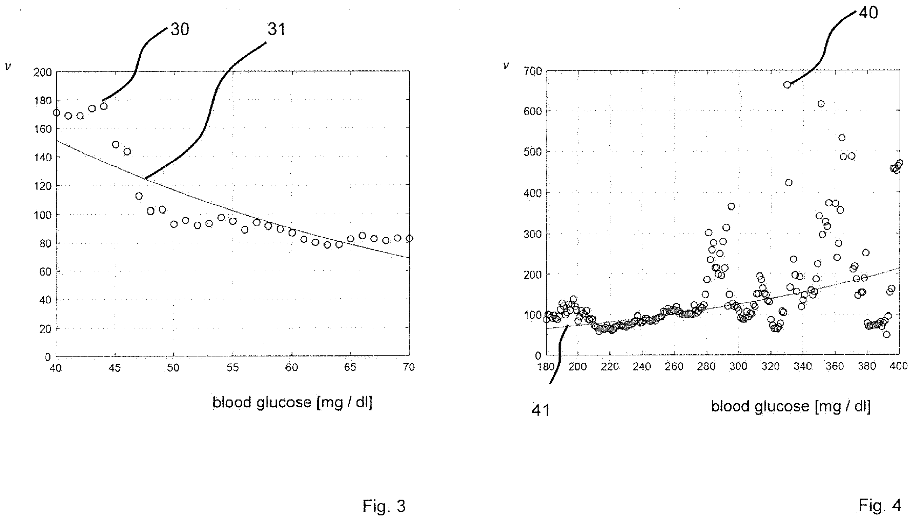

Subsequently, a Gaussian distribution is then fit for each class, yielding continuous kernel bandwidth values 31, 41, in order to determine the kernel bandwidth for any glucose within the class. Fig. 3 shows a graphical representation of the discrete kernel bandwidth values 30 together with a fitting function 31 in the hypoglycemia class, whereas Fig. 4 shows a graphical representation the discrete kernel bandwidth values 40 together with a fitting function 41 in the hyperglycemia class. The respective x-axes correspond to blood glucose values g, the y-axes correspond to kernel bandwidth values v.

The discrete kernel bandwidth values 40 exhibit a higher degree of noise for larger blood glucose values g. For the hypoglycemia class, the fitting function 31 for the discrete kernel bandwidth values 30 has the formula ${ν}_{1} \left(g\right) = \frac{1}{60} {e}^{6.06895 - 0.02620 g} ,$

For the hyperglycemia class, the fitting function 41 for the discrete kernel bandwidth values 40 has the formula ${ν}_{3} \left(g\right) = \frac{1}{60} {e}^{3.23307 + 0.00532 g} .$

For the non-adverse blood glucose range class, the kernel bandwidth is constant (*ν*₂).

In order to determine the kernel bandwidth v across the entire range of blood glucose values, the fitting functions are combined: $ν \left(g\right) = \left\{\begin{matrix}{ν}_{1} \left(g\right) & g \leq 70 mg / dl \\ {ν}_{2} & 70 mg / dl < g < 180 mg / dl \\ {ν}_{3} \left(g\right) & 180 mg / dl \leq g\end{matrix}\right.$

With the kernel bandwidth being dependent on the blood glucose values, the resulting estimator *f̂* for the PDF *f* has the modified formula $\hat{f} \left(θ\right) = {\sum }_{i = 1}^{n} \frac{1}{n \left(2π\right) {I}_{0} \left({ν}_{i}\right)} {e}^{{ν}_{i} cos \left(θ-{θ}_{i}\right)} .$

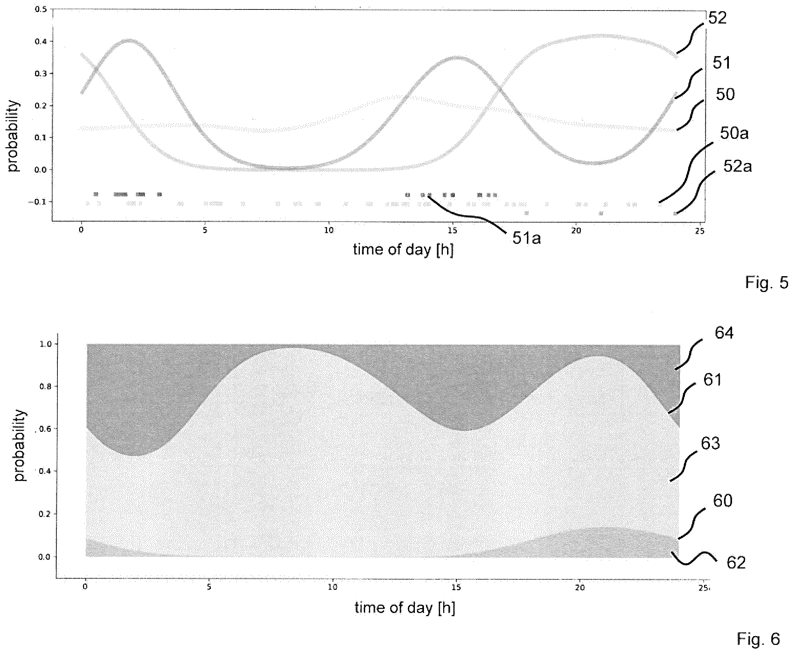

Fig. 5 shows a graphical representation of determined PDFs 50, 51, 52 which are determined from discrete BGM values 50a, 51a, 52a, respectively.

The x-axis represents the time of day in unit of hours from 0 to 24. The PDFs 50, 51, 52 are not yet normalized. The PDF 50 and the blood glucose measurement values 50a correspond to a non-adverse blood glucose state, the PDF 51 and the blood glucose measurement values 51a to a hyperglycemic state, and the PDF 52 and the blood glucose measurement values 52a to a hypoglycemic state. The PDF 50 has been determined from the blood glucose measurement values 50a assigned to the non-adverse blood glucose state. The PDF 51 has been determined from the blood glucose measurement values 51a to the hyperglycemic state. Further, the PDF 52 has been determined from the blood glucose measurement values 52a to a hypoglycemic state.

As can be seen from Fig. 5, the appearance of blood glucose measurement values assigned to a hypoglycemic and a hyperglycemic state at certain times of day coincides with larger values of the corresponding PDF at those times of day.

Fig. 6 shows a graphical representation of the probabilities of being in a hyperglycemic state, a hypoglycemic state, or a non-adverse blood glucose state for each time of day in unit of hour from 0 to 24. Curve 60 corresponds to the PDF 52 after normalization and curve 61 to PDF 52 after normalization plus PDF 50 after normalization. Area 62, which is below the curve 60, represents the probability of being in a hypoglycemic state for a certain time of day. Area 63, which is between the curves 60 and 61, represents the probability of being in a non-adverse blood glucose state for a certain time of day. Area 64, which is above the curve 61, represents the probability of being in a hyperglycemic state for a certain time of day.

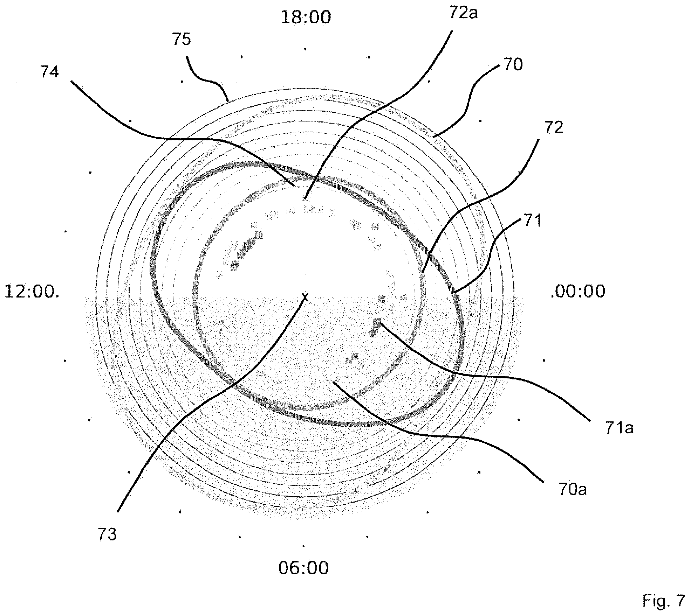

The periodicity of the time of day can be illustrated more adequately in a polar diagram. Hereto, Fig. 7 shows a polar representation of the determined PDFs 50, 51, 52 after normalization. The PDFs 70, 71, and 72 correspond to the PDFs 50, 51, and 52, respectively. The times of day from 0 to 24 hours correspond to angles from 0 to 2*π* (clockwise). Increasing radial distance from the origin 73 corresponds to a higher value of the PDFs 70, 71, and 72. Circle 74 corresponds to a PDF value of 0; circle 75 to a PDF value of 1. Blood glucose measurement values 70a, 71a, and 72a correspond to the blood glucose measurement values 50a, 51a, and 52a, respectively.

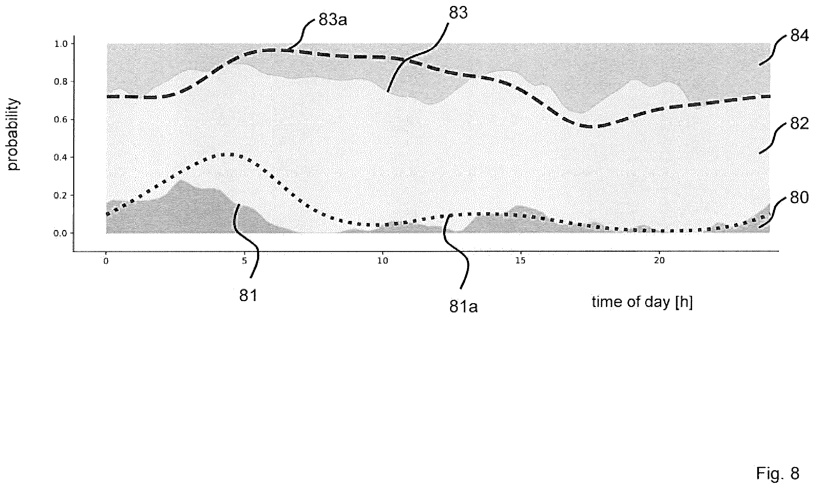

Fig. 8 shows a graphical representation of the probabilities of being in a hyperglycemic state, a hypoglycemic state, or a non-adverse blood glucose state determined from discrete BGM values in comparison with the probabilities determined from a CGM data set. Area 80, which is below curve 81, represents the probability of being in a hypoglycemic state for a certain time of day determined from discrete BGM values. Area 82, which is between the curve 81 and curve 83, represents the probability of being in a non-adverse blood glucose state for a certain time of day determined from discrete BGM values. Area 84, which is above the curve 83, represents the probability of being in a hyperglycemic state for a certain time of day determined from discrete BGM values. Correspondingly, the area below curve 81a represents the probability of being in a hypoglycemic state for a certain time of day determined from CGM values; the area between the curve 81a and curve 83a represents the probability of being in a non-adverse blood glucose state for a certain time of day determined from CGM values; and the area above the curve 83a represents the probability of being in a hyperglycemic state for a certain time of day determined from CGM values.

Model performance can be assessed by comparing the probabilities using the entire CGM data set with the determined probabilities from subsets of the CGM data set simulating BGM use cases. To this end, CGM data sets of 36 patients for a period of 14 days were employed, yielding ground truth PDFs. Subsequently, kernel bandwidths were calculated and PDFs determined using subsamples of the CGM data set with varying number of measurements per day. The resulting determined PDFs were compared to the ground truth PDFs via relative residual sums of squared errors (RSS).

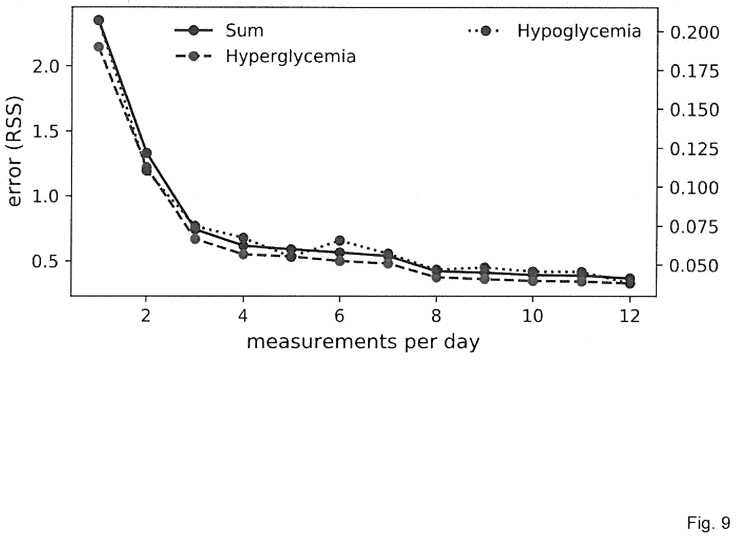

This is illustrated by Fig. 9, which shows a graphical representation of the RSS error as a function of the employed number of measurements per day. As the number of measurements per day increases, the RSS error decreases. The model performance reaches a plateau at approximately four measurements per day.

In another example, uncertainty in determining classes can be taken into account by including an additional or further class corresponding to unspecified or unknown states. As more blood glucose measurement values are included, the influence of the unspecified state class is reduced. The uncertainty is represented by data that is uniformly extended over a day. The number of data points of this class can be adjusted to control the impact that a single blood glucose measurement has towards the determined PDF. Thus, the influence of outlier events on determining PDFs can be controlled.

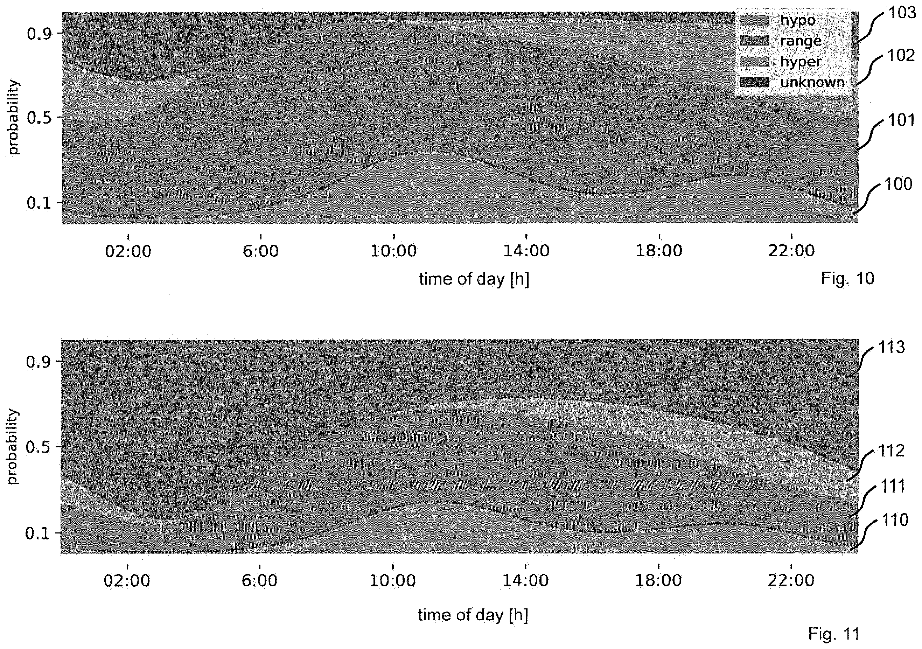

Figs. 10 and 11 show graphical representations of the probabilities of being in a hypoglycemic state 100, 110, a non-adverse blood glucose state 101, 111, a hyperglycemic state 102, 112, or an unspecified or unknown state 103, 113 (further range of probability for blood glucose), respectively, determined from discrete BGM values. Less data were measured by the patient during night time. Consequently, the determined probabilities during the night time base on less evidence, which is indicated by larger probabilities of being in the unspecified or unknown state 103, 113 in particular for a time of day from 1 a.m. to 3 a.m. Decreasing the size of the blood glucose measurement data employed for determining the probabilities results in an increase of the probability of being in an unspecified state. The size of the employed blood glucose measurement data in the example according to Fig. 10 is larger than in the example according to Fig. 11. Correspondingly, the area corresponding to the unspecified state 103 is smaller than the area corresponding to the unspecified state 113.

## Claims

1. A method of determining a probability of a blood glucose value for a patient being in an adverse blood glucose range at a prediction time, the method, in a system (10) having one or more data processors, comprising:
- providing spot monitoring blood glucose measurement data representing a plurality of blood glucose measurement values for a measurement time period, wherein the blood glucose measurement values comprise
- first blood glucose measurement values assigned to a first adverse range of blood glucose values; and
- second blood glucose measurement values assigned to a second adverse range of blood glucose values, wherein the second range of blood glucose values is different from the first adverse range of blood glucose values;
**characterized by**
- by applying an analysis algorithm comprising a kernel density estimation and application of Bayes' rule, determining from the spot monitoring blood glucose measurement data comprising the first blood glucose measurement values and the second blood glucose measurement values a probability of
- the blood glucose value of the patient being in the first adverse blood glucose range at the prediction time, and
- the blood glucose value of the patient being in the second adverse blood glucose range at the prediction time;
wherein, in the kernel density estimation, a first kernel bandwidth is applied for all or some of the first blood glucose measurement values and a second kernel bandwidth different from the first kernel bandwidth is applied for all or some of the second blood glucose measurement values; and
- providing output data indicative of the prediction time and the probability at the prediction time.

2. Method according to claim 1, wherein the probability is determined at a plurality of prediction times in a prediction period of time from the spot monitoring blood glucose measurement data.

3. Method according to claim 2, wherein a continuous course of the probability is determined at a plurality of prediction times in the prediction period of time from the spot monitoring blood glucose measurement data.

4. Method according to any claim of the preceding claims, wherein the applying of the analysis algorithm comprises
- determining the probability of the blood glucose value of the patient being in the first adverse blood glucose range at the prediction time from a first measurement data subset of the spot monitoring blood glucose measurement data comprising at least the first blood glucose measurement values assigned to the first adverse range of blood glucose values; and
- determining the probability of the blood glucose value of the patient being in the second adverse blood glucose range at the prediction time from a second measurement data subset of the spot monitoring blood glucose measurement data comprising at least the second blood glucose measurement values assigned to the second adverse range of blood glucose values.

5. Method according to any claim of the preceding claims, wherein the determining of the probability comprises determining a probability for the blood glucose value of the patient being in a non-adverse blood glucose range at the prediction time, wherein the non-adverse blood glucose range is different from the first and second adverse blood glucose range.

6. Method according to claim 5, wherein the determining further comprises applying a third kernel bandwidth in the kernel density estimation which is different from both the first and the second kernel bandwidth.

7. Method according to any claim of the preceding claims, wherein the applying comprises applying a periodic kernel in the kernel density estimation.

8. Method according to any claim of the preceding claims, wherein the first kernel bandwidth is broader than the second kernel bandwidth.

9. Method according to any claim of the preceding claims, wherein the applying comprises
- applying a first bandwidth value for a measurement value from the first blood glucose measurement values; and
- applying a second bandwidth value for a further measurement value from the second blood glucose measurement values,
wherein the first bandwidth value is different from the second bandwidth value.

10. Method according to any claim of the preceding claims, wherein
- the first adverse range of blood glucose values is assigned to blood glucose measurement values indicative of a hypoglycemic state for the patient; and
- the second adverse range of blood glucose values is assigned to blood glucose measurement values indicative of a hyperglycemic state for the patient.

11. A system (10) for determining a probability of a blood glucose value for a patient being in an adverse blood glucose range at a prediction time, the system (10) having one or more data processors, and the one or more data processors configured to perform the method according to at least one of the claims 1 to 10.

12. Computer program product, comprising program code configured to, when loaded into a computer having one or more processors, perform the method according to at least one of the claims 1 to10.

## Patentansprüche

1. Verfahren zur Bestimmung der Wahrscheinlichkeit eines Blutzuckerwerts für einen Patienten, der sich zu einer Vorhersagezeit in einem unerwünschten Blutzuckerbereich befindet, wobei das Verfahren in einem System (10) mit einem oder mehreren Datenprozessoren Folgendes umfasst:
- Bereitstellen von Punktüberwachungs-Blutzuckermessdaten, die eine Vielzahl von Blutzuckermesswerten für einen Messzeitraum darstellen, wobei die Blutzuckermesswerte Folgendes umfassen:
- erste Blutzuckermesswerte, die einem ersten unerwünschten Bereich von Blutzuckerwerten zugeordnet sind; und
- zweite Blutzuckermesswerte, die einem zweiten unerwünschten Bereich von Blutzuckerwerten zugeordnet sind, wobei sich der zweite Bereich von Blutzuckerwerten von dem ersten unerwünschten Bereich von Blutzuckerwerten unterscheidet;
**dadurch gekennzeichnet, dass**
- aus den Punktüberwachungs-Blutzuckermessdaten, die die ersten Blutzuckermesswerte und die zweiten Blutzuckermesswerte umfassen, durch Anwenden eines Analysealgorithmus, der eine Kerndichteschätzung und die Anwendung der Bayes'schen Regel umfasst, die Wahrscheinlichkeit bestimmt wird, dass
- sich der Blutzuckerwert des Patienten zu der Vorhersagezeit in dem ersten unerwünschten Blutzuckerbereich befindet und
- sich der Blutzuckerwert des Patienten zu der Vorhersagezeit in dem zweiten unerwünschten Blutzuckerbereich befindet;
wobei bei der Kerndichteschätzung eine erste Kernbandbreite für alle oder einige der ersten Blutzuckermesswerte angewendet wird und eine zweite Kernbandbreite, die sich von der ersten Kernbandbreite unterscheidet, für alle oder einige der zweiten Blutzuckermesswerte angewendet wird; und
- Bereitstellen von Ausgabedaten, die die Vorhersagezeit und die Wahrscheinlichkeit zu der Vorhersagezeit angeben.

2. Verfahren nach Anspruch 1, wobei die Wahrscheinlichkeit zu einer Vielzahl von Vorhersagezeiten in einem Vorhersagezeitraum aus den Punktüberwachungs-Blutzuckermessdaten bestimmt wird.

3. Verfahren nach Anspruch 2, wobei ein kontinuierlicher Verlauf der Wahrscheinlichkeit zu einer Vielzahl von Vorhersagezeiten in dem Vorhersagezeitraum aus den Punktüberwachungs-Blutzuckermessdaten bestimmt wird.

4. Verfahren nach einem der vorhergehenden Ansprüche, wobei das Anwenden des Analysealgorithmus Folgendes umfasst:
- Bestimmen der Wahrscheinlichkeit, dass sich der Blutzuckerwert des Patienten zu der Vorhersagezeit in dem ersten unerwünschten Blutzuckerbereich befindet, aus einer ersten Messdatenteilmenge der Punktüberwachungs-Blutzuckermessdaten, die mindestens die ersten Blutzuckermesswerte umfassen, die dem ersten unerwünschten Bereich von Blutzuckerwerten zugeordnet sind; und
- Bestimmen der Wahrscheinlichkeit, dass sich der Blutzuckerwert des Patienten zu der Vorhersagezeit in dem zweiten unerwünschten Blutzuckerbereich befindet, aus einer zweiten Messdatenteilmenge der Punktüberwachungs-Blutzuckermessdaten, die mindestens die zweiten Blutzuckermesswerte umfassen, die dem zweiten unerwünschten Bereich von Blutzuckerwerten zugeordnet sind.

5. Verfahren nach einem der vorhergehenden Ansprüche, wobei das Bestimmen der Wahrscheinlichkeit das Bestimmen einer Wahrscheinlichkeit umfasst, dass sich der Blutzuckerwert des Patienten zu der Vorhersagezeit in einem nicht unerwünschten Blutzuckerbereich befindet, wobei sich der nicht unerwünschte Blutzuckerbereich von dem ersten und zweiten unerwünschten Blutzuckerbereich unterscheidet.

6. Verfahren nach Anspruch 5, wobei das Bestimmen ferner das Anwenden einer dritten Kernbandbreite bei der Kerndichteschätzung umfasst, die sich sowohl von der ersten als auch der zweiten Kernbandbreite unterscheidet.

7. Verfahren nach einem der vorhergehenden Ansprüche, wobei das Anwenden das Anwenden eines periodischen Kerns bei der Kerndichteschätzung umfasst.

8. Verfahren nach einem der vorhergehenden Ansprüche, wobei die erste Kernbandbreite breiter als die zweite Kernbandbreite ist.

9. Verfahren nach einem der vorhergehenden Ansprüche, wobei das Anwenden Folgendes umfasst:
- Anwenden eines ersten Bandbreitenwerts für einen Messwert aus den ersten Blutzuckermesswerten; und
- Anwenden eines zweiten Bandbreitenwerts für einen weiteren Messwert aus den zweiten Blutzuckermesswerten,
wobei sich der erste Bandbreitenwert von dem zweiten Bandbreitenwert unterscheidet.

10. Verfahren nach einem der vorhergehenden Ansprüche, wobei
- der erste unerwünschte Bereich von Blutzuckerwerten Blutzuckermesswerten zugeordnet ist, die einen hypoglykämischen Zustand für den Patienten angeben; und
- der zweite unerwünschte Bereich von Blutzuckerwerten Blutzuckermesswerten zugeordnet ist, die einen hyperglykämischen Zustand für den Patienten angeben.

11. System (10) zur Bestimmung der Wahrscheinlichkeit eines Blutzuckerwerts für einen Patienten, der sich zu einer Vorhersagezeit in einem unerwünschten Blutzuckerbereich befindet, wobei das System (10) einen oder mehrere Datenprozessoren aufweist und der eine oder die mehreren Datenprozessoren eingerichtet sind, das Verfahren nach mindestens einem der Ansprüche 1 bis 10 durchzuführen.

12. Computerprogrammprodukt, umfassend einen Programmcode, der eingerichtet ist, das Verfahren nach mindestens einem der Ansprüche 1 bis 10 durchzuführen, wenn dieser in einen Computer mit einem oder mehreren Prozessoren geladen wird.

## Revendications

1. Procédé permettant de déterminer la probabilité qu'une valeur de glucose sanguin d'un patient se trouve dans une plage de glucose sanguin défavorable à un moment de prédiction, le procédé, dans un système (10) ayant un ou plusieurs processeurs de données, comprenant :
- la fourniture de données de mesure de glucose sanguin de surveillance ponctuelle représentant une pluralité de valeurs de mesure de glucose sanguin pour une période de mesure, dans lequel les valeurs de mesure de glucose sanguin comprennent
- des premières valeurs de mesure de glucose sanguin attribuées à une première plage de valeurs de glucose sanguin défavorable ; et
- des secondes valeurs de mesure de glucose sanguin attribuées à une seconde plage de valeurs de glucose sanguin défavorable, dans lequel la seconde plage de valeurs de glucose sanguin est différente de la première plage de valeurs de glucose sanguin défavorable ;
**caractérisé par**
- en appliquant un algorithme d'analyse comprenant une estimation de densité par noyau et une application de la règle de Bayes, la détermination à partir des données de mesure de glucose sanguin de surveillance ponctuelle comprenant les premières valeurs de mesure de glucose sanguin et les secondes valeurs de mesure de glucose sanguin de la probabilité que
- la valeur de glucose sanguin du patient se trouve dans la première plage de glucose sanguin défavorable au moment de prédiction, et
- la valeur de glucose sanguin du patient se trouve dans la seconde plage de glucose sanguin défavorable au moment de prédiction ;
dans lequel, dans l'estimation de densité par noyau, une première bande passante de noyau est appliquée pour toutes ou certaines des premières valeurs de mesure de glucose sanguin et une deuxième bande passante de noyau différente de la première bande passante de noyau est appliquée à toutes ou certaines des secondes valeurs de mesure de glucose sanguin ; et
- la fourniture de données de sortie indicatives du moment de prédiction et de la probabilité au moment de prédiction.

2. Procédé selon la revendication 1, dans lequel la probabilité est déterminée à une pluralité de moments de prédiction dans une période de prédiction à partir des données de mesure de glucose sanguin de surveillance ponctuelle.

3. Procédé selon la revendication 2, dans lequel une évolution continue de la probabilité est déterminée à une pluralité de moments de prédiction dans la période de prédiction à partir des données de mesure de glucose sanguin de surveillance ponctuelle.

4. Procédé selon une revendication quelconque des revendications précédentes, dans lequel l'application de l'algorithme d'analyse comprend
- la détermination de la probabilité que la valeur de glucose sanguin du patient se trouve dans la première plage de glucose sanguin défavorable au moment de prédiction à partir d'un premier sous-ensemble de données de mesure des données de mesure de glucose sanguin de surveillance ponctuelle comprenant au moins les premières valeurs de mesure de glucose sanguin attribuées à la première plage de valeurs de glucose sanguin défavorable ; et
- la détermination de la probabilité que la valeur de glucose sanguin du patient se trouve dans la seconde plage de glucose sanguin défavorable au moment de prédiction à partir d'un second sous-ensemble de données de mesure des données de mesure de glucose sanguin de surveillance ponctuelle comprenant au moins les secondes valeurs de mesure de glucose sanguin attribuées à la seconde plage de valeurs de glucose sanguin défavorable.

5. Procédé selon une revendication quelconque des revendications précédentes, dans lequel la détermination de la probabilité comprend la détermination de la probabilité que la valeur de glucose sanguin du patient se trouve dans une plage de glucose sanguin non défavorable au moment de prédiction, dans lequel la plage de glucose sanguin non défavorable est différente de la première et de la seconde plage de glucose sanguin défavorable.

6. Procédé selon la revendication 5, dans lequel la détermination comprend en outre l'application d'une troisième bande passante de noyau dans l'estimation de densité par noyau qui est différente à la fois de la première et de la deuxième bande passante de noyau.

7. Procédé selon une revendication quelconque des revendications précédentes, dans lequel l'application comprend l'application d'un noyau périodique dans l'estimation de densité par noyau.

8. Procédé selon une revendication quelconque des revendications précédentes, dans lequel la première bande passante de noyau est plus large que la deuxième bande passante de noyau.

9. Procédé selon une revendication quelconque des revendications précédentes, dans lequel l'application comprend
- l'application d'une première valeur de bande passante pour une valeur de mesure à partir des premières valeurs de mesure de glucose sanguin ; et
- l'application d'une seconde valeur de bande passante pour une valeur de mesure supplémentaire à partir des secondes valeurs de mesure de glucose sanguin,
dans lequel la première valeur de bande passante est différente de la seconde valeur de bande passante.

10. Procédé selon une revendication quelconque des revendications précédentes, dans lequel
- la première plage de valeurs de glucose sanguin défavorable est attribuée à des valeurs de mesure de glucose sanguin indicatives d'un état hypoglycémique du patient ; et
- la seconde plage de valeurs de glucose sanguin défavorable est attribuée à des valeurs de mesure de glucose sanguin indicatives d'un état hyperglycémique du patient.

11. Système (10) permettant de déterminer la probabilité qu'une valeur de glucose sanguin d'un patient se trouve dans une plage de glucose sanguin défavorable à un moment de prédiction, le système (10) ayant un ou plusieurs processeurs de données, et le ou les processeurs de données étant configurés pour réaliser le procédé selon au moins l'une des revendications 1 à 10.

12. Produit-programme d'ordinateur, comprenant un code de programme configuré pour, lorsqu'il est chargé dans un ordinateur ayant un ou plusieurs processeurs, réaliser le procédé selon au moins l'une des revendications 1 à 10.
